# EUROPÄISCHE PATENTSCHRIFT

(11) **EP 1 438 934 B1**
(45) Veröffentlichungstag und Bekanntmachung des Hinweises auf die Patenterteilung: **06.07.2005**
(21) Anmeldenummer: 03023282.1
(22) Anmeldetag: 15.10.2003
(51) Int. Cl.: A61F 2/36

(54) **Set zur Erstellung eines Gelenkkopf-Kappenimplantates für ein künstliches Hüftgelenk**
Set for constructing a cup shaped joint head implant for an artificial hip joint
Ensemble pour la construction d'un implant de tête d'articulation de forme evasée pour une articulation de hanche artificielle

(30) Priorität: 15.01.2003 DE 10301986
(43) Veröffentlichungstag der Anmeldung: 21.07.2004
(73) Patentinhaber: ESKA Implants GmbH & Co., 23556 Lübeck (DE)
(72) Erfinder: Grundei, Hans, Dr.-Ing., 23558 Lübeck (DE); Thomas, Wolfram, Prof. Dr. med., 00135 Roma (IT)
(74) Vertreter: Fuchs Mehler Weiss & Fritzsche

(56) Entgegenhaltungen:
- EP-A- 0 878 176
- EP-A- 1 004 276
- EP-A- 1 260 200
- DE-A- 10 007 615
- US-A- 3 064 645

## Beschreibung

Die vorliegende Erfindung betrifft ein Set zur Erstellung eines Gelenkkopf-Kappenimplantates für ein künstliches Hüftgelenk, wie aus dem Dokument EP-A-1 260 200 bekannt.

In jüngster Zeit kommen verstärkt sogenannte Kappenimplantate zur Anwendung, welche über den präparierten natürlichen Restgelenkkopf bzw. über den resezierten Schenkelkopfhals gesetzt werden und in dieser Lage dann fixiert werden. Voraussetzung für eine stabile Sekundärfixation ist stabiles Knochenmaterial des Restknochens. So wird gemäß der DE-A-102 18 801 vorgeschlagen, an die Gelenkkopfkappe einen Zapfen anzukoppeln, der in eine entsprechende Ausfräsung im Schenkelhals gesetzt wird. Dieser Zapfen weist eine Oberfläche auf, welche mit einer dreidimensionalen offenmaschigen Raumnetzstruktur versehen ist, in welche und durch welche hindurch Knochentrapekel des umliegende Knochenmaterials wachsen und für die stabile Sekundärfixation sorgen.

Diese Patientenversorgung scheitert bei geschwächtem Knochenmaterial, beispielsweise bei Osteoporose. Insbesondere ist davon auszugehen, dass mehr weibliche Patientinnen hiervon betroffen sind als männliche Patienten, so dass es die Aufgabe der vorliegenden Erfindung ist, ein Set der eingangs genannten Art so weiter zu entwickeln, dass eine stabile Sekundärfixation auch im Falle geschwächten Knochenmaterials im Restknochen gewährleistet wird.

Gelöst wird diese Aufgabe durch ein Set, welches wenigstens eine der Form der natürlichen Gelenkkugel nachgebildeten Kappe, die auf einen resezierten Schenkelkopfhals setzbar ist, sowie eine wenigstens Fixationshilfe aufweist, welche proximal mit der Kappe verbindbar ist und aus einem dünnwandigen hohlen Armierungskörper mit gekrümmtem Verlauf besteht von solcher Länge, dass er durch den Schenkelhals in den Knochenkanal im Femur führbar und entlang des Knochenkanals fixierbar ist, wobei die Oberfläche des Armierungskörpers durch eine Vielzahl von Durchbrechungen durchsetzt ist.

In den Armierungskörper können die Knochentrapekel des umgebenden Knochenmaterials einheilen und so für eine stabile Dauerfixation sorgen. Die Krümmung des Armierungskörpers ist besonders bevorzugt so gewählt, dass der Armierungskörper in situ in etwa dem Adam'schen Bogen des Femurs im Bereich des Trochanter minors folgt. Dies führt zu einer besonders günstigen Belastungsverteilung im Knochenmaterial.

Der Armierungskörper wirkt vorliegend als stabilisierendes Element, hat jedoch darüber hinaus die Wirkung eines Prophylaxe-Implantates, wie es beispielsweise bekannt ist aus der DE-A-100 07 615. Allerdings steht vorliegend nicht der prophylaktische Charakter des Armierungskörpers im Vordergrund, sondern vielmehr der funktionelle, insofern, als dass vorliegend der Armierungskörper tatsächlich Ablastfunktionen übernimmt, was bei einem bekannten Prophylaxe-Implantat nicht der Fall ist.

Bevorzugt ist der Armierungskörper gebildet aus einem zu einem Rohr geformten Gitternetzwerk. Das Gitternetzwerk gewährleistet eine hinreichende Anzahl von Durchbrechungen, durch welche die Knochentrapekel in das Innere des Armierungskörpers wachsen können.

Eine alternative Ausführungsform des Armierungskörpers als Teil eines Sets gemäß einem zweiten Lösungsvorschlag unterscheidet sich von dem vorbeschriebenen Armierungskörper dadurch, dass er aus einem Hohlrohr besteht, dessen Außenwandung gebildet ist aus einer offenmaschigen dreidimensionalen Raumnetzstruktur. Durch diese Raumnetzstruktur kann Knochenmaterial von Außen in das Innere der Hülse wachsen. Darüber hinaus bietet diese Raumnetzstruktur eine Primärfixation des Implantates im Femur.

Für die Fixation der Kappe auf dem Schenkelhals ist vorteilhaft vorgesehen, dass der Boden der Kappe mit ebenfalls der offenmaschigen dreidimensionalen Raumnetzstruktur versehen ist. Die Maschigkeiten der Raumnetzstrukturen auf dem Armierungskörper gemäß dem zweiten Lösungsvorschlag und an dem Boden der Kappe können durchaus unterschiedlich sein und den Größen der Knochentrapekel angepasst sein.

Die Verbindung zwischen der Kappe und dem Armierungskörper wird vorteilhaft mittels einer selbsthemmenden Schraubverbindung realisiert, die gewährleistet, dass die Kappe mit dem Armierungskörper rotationsgesichert verbunden bleibt.

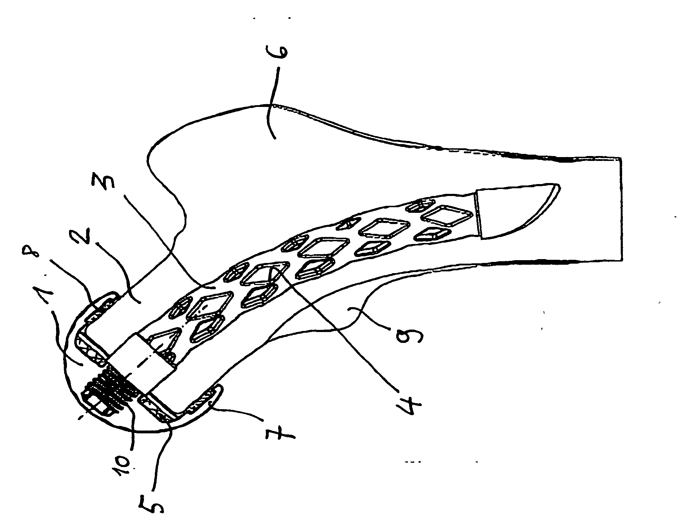

Die Erfindung wird anhand eines Ausführungsbeispiels gemäß der einzigen Zeichnungsfigur näher erläutet. Diese zeigt ein aus dem erfindungsgemäßen Set erstelltes Gelenkkopf-Kappenimplantat in situ im Femur implantiert.

Der Femur (6) weist einen resezierten Schenkelhals (2) auf. Die natürliche Gelenkkugel wurde vor der Implantation reseziert. Die Funktion nimmt nun eine der Form der natürlichen Gelenkkugel nachgebildeten Kappe (1) wahr. Diese ist auf den Schenkelhals (2) gesetzt und fasst diesen mittels eines umlaufenden Bundes (7) ein. Der Boden der Kappe (1) ist vorliegend versehen mit einer Schicht einer offenmaschigen dreidimensionalen Raumnetzstruktur (5), in die und durch welche hindurch Knochentrapekel, die frontal aus dem resezierten Schenkelhals (2) wachsen, einheilen. Ähnlich belegt ist im Inneren der umlaufende Bund (7) mit einer offenmaschigen dreidimensionalen Raumnetzstruktur (8). Die Raumnetzstrukturen am Boden (5) und im umlaufenden Bund (7) dienen zur Langzeitfixation der Kappe auf dem Schenkelhals (2).

Mit der Kappe (1) verbunden ist ein hohler Armierungskörper (3). Dieser Armierungskörper (3) weist einen gekrümmten Verlauf auf, der annähernd dem Adam'schen Bogen im Bereich des Trochanter minors (9) folgt.

Der Armierungskörper (3) besteht vorliegend aus einem zu einem Rohr geformten Gitternetzwerk, welches eine Vielzahl von Durchbrechungen (4) in seiner Außenwandung aufweist, durch welche hindurch Knochentrapekel des umliegenden Knochenmaterials wachsen können und so zur stabilen Sekundärfixation beitragen bzw. diese erst ermöglichen.

Die stabile Verbindung zwischen der Kappe (1) und dem Armierungskörper (3) ist vorliegend mittels einer selbsthemmenden Schraubverbindung (10) realisiert. Der Operateur wählt nach Erfassung und Vermessung der gegebenen Größenverhältnisse patientenindividuell aus dem Set eine Kappe (1) und ein Armierungskörper (3) aus und fügt diese so zusammen, dass er eine selbsthemmende Schraubverbindung zwischen den beiden Teilen herstellt.

## Patentansprüche

1. Set zur Erstellung eines Gelenkkopf-Kappenimplantates für ein künstliches Hüftgelenk, aufweisend wenigstens eine der Form der natürlichen Gelenkkugel nachgebildeten Kappe (1), die auf einen resezierten Schenkelkopfhals (2) setzbar ist, sowie wenigstens eine Fixationshilfe, welche proximal mit der Kappe (1) verbindbar ist und aus einem dünnwandigen hohlen Armierungskörper (3) mit gekrümmtem Verlauf besteht von solcher Länge, dass er durch den Schenkelhals (2) in einen Knochenkanal im Femur führbar und entlang des Knochenkanals fixierbar ist, wobei die Oberfläche des Armierungskörpers (3) durch eine Vielzahl von Durchbrechungen (4) durchsetzt ist.

2. Set nach Anspruch 1, bei dem der Armierungskörper (3) aus einem zu einem Rohr geformten Gitternetzwerk gebildet ist.

3. Set zur Erstellung eines Gelenkkopf-Kappenimplantates für ein künstliches Hüftgelenk, aufweisend wenigstens eine der Form der natürlichen Gelenkkugel nachgebildeten Kappe (1), die auf einen resezierten Schenkelkopfhals (2) setzbar ist, sowie wenigstens eine Fixationshilfe, welche proximal mit der Kappe (1) verbindbar ist und aus einem Hohlrohr als Armierungskörper (3) besteht, dessen Außenwandung gebildet ist aus einer offenmaschigen dreidimensionalen Raumnetzstruktur, mit einem gekrümmten Verlauf und von solcher Länge, dass er durch den Schenkelhals in einen Knochenkanal im Femur führbar und entlang dieses Knochenkanals fixierbar ist.

4. Set nach einem der Ansprüche 1 bis 3, bei dem der Boden der Kappe (1) mit einer offenmaschigen dreidimensionalen Raumnetzstruktur (5) versehen ist.

5. Set nach einem der Ansprüche 1 bis 4, bei dem die Verbindung zwischen Kappe und Fixationshilfe mittels einer Selbsthemmenden Schraubverbindung (10) realisierbar ist.

## Claims

1. Set for constructing a condyle cap implant for an artificial hip joint, having at least one cap (1) copying the shape of the natural condyle and which can be placed on a resected femoral neck (2), and at least one fixing aid which can be connected proximally to the cap (1) and consists of a thin-walled hollow reinforcing body (3) with curved shape of such length that it can be guided through the femoral neck (2) into a bone channel in the femur and can be fixed along the bone channel, wherein the surface of the reinforcing body (3) is penetrated by a plurality of apertures (4).

2. Set according to claim 1, in which the reinforcing body (3) is formed from a lattice network shaped to form a tube.

3. Set for constructing a condyle cap implant for an artificial hip joint, having at least one cap (1) copying the shape of the natural condyle and which can be placed on a resected femoral neck (2), and at least one fixing aid which can be connected proximally to the cap (1) and consists of a hollow tube as reinforcing body (3), the outer wall of which is formed from an open-mesh three-dimensional spatial network structure, with a curved shape and of such length that it can be guided through the femoral neck into a bone channel in the femur and can be fixed along this bone channel.

4. Set according to one of claims 1 to 3, in which the base of the cap (1) is provided with an open-mesh three-dimensional spatial network structure (5).

5. Set according to one of claims 1 to 4, in which the connection between cap and fixing aid can be realised by means of a self-locking screw connection (10).

## Revendications

1. Ensemble pour la construction d'un implant de tête d'articulation de forme évasée pour une articulation de hanche artificielle, présentant au moins une cupule (1) reproduite d'après la forme d'une sphère d'articulation naturelle, qui peut être placée sur une tête de col de fémur réséquée (2), et au moins un accessoire de fixation, qui peut être relié de manière proximale à la cupule (1) et se compose d'un élément d'armature creux à paroi mince (3) avec un tracé courbe, d'une longueur telle que, à travers le col du fémur (2), il peut être introduit dans le fémur dans un canal osseux et être fixé le long du canal osseux sachant que la surface de l'élément d'armature (3) contient une pluralité d'encoches (4).

2. Ensemble selon la revendication 1, dans lequel l'élément d'armature (3) est formé d'un réseau quadrillé en forme de tuyau.

3. Ensemble pour la construction d'un implant de tête d'articulation de forme évasée pour une articulation de hanche artificielle, présentant au moins une cupule (1) reproduite d'après la forme d'une sphère d'articulation naturelle, qui peut être placée sur une tête de col de fémur réséquée (2), et au moins un accessoire de fixation, qui peut être relié de manière proximale à la cupule (1) et se compose d'un tuyau creux servant d'élément d'armature (3) dont la paroi extérieure est formée d'une structure en réseau à mailles ouvertes en trois dimensions, avec un tracé courbe et d'une longueur telle que, à travers le col du fémur (2), il peut être introduit dans le fémur dans un canal osseux et être fixé le long de ce canal.

4. Ensemble selon l'une des revendications 1 à 3, dans lequel le fond de la cupule (1) est prévu avec une structure en réseau à mailles ouvertes en trois dimensions (5).

5. Ensemble selon l'une des revendications 1 à 4, dans lequel la liaison entre la cupule et l'accessoire de fixation peut être réalisée au moyen d'un assemblage par vis à blocage automatique (10).
